(19) Europäisches Patentamt
European Patent Office
Office européen des brevets

(11) **EP 3 554 361 B1**

(12) **EUROPEAN PATENT SPECIFICATION**

(45) Date of publication and mention
of the grant of the patent:
**29.09.2021 Bulletin 2021/39**

(51) Int Cl.:
***A61B 5/369*** *(2021.01)*

(21) Application number: **17825465.2**

(22) Date of filing: **15.12.2017**

(86) International application number:
**PCT/EP2017/083027**

(87) International publication number:
**WO 2018/109166 (21.06.2018 Gazette 2018/25)**

(54) **IMPROVED SIGNAL QUALITY INDEX OF MULTICHANNEL BIO-SIGNAL USING RIEMANNIAN GEOMETRY**

VERBESSERTER SIGNALQUALITÄTSINDEX VON MEHRKANAL-BIOSIGNAL MIT RIEMANNSCHER GEOMETRIE

INDICE DE QUALITÉ DE SIGNAL AMÉLIORÉE DE BIO-SIGNAL MULTICANAL UTILISANT LA GÉOMÉTRIE DE RIEMANN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.12.2016 EP 16204486**

(43) Date of publication of application:
**23.10.2019 Bulletin 2019/43**

(73) Proprietor: **CYREBRO Technologies**
**75010 Paris (FR)**

(72) Inventors:
• **BARTHELEMY, Quentin**
**69003 Lyon (FR)**
• **MAYAUD, Louis**
**16000 Angoulême (FR)**

(74) Representative: **Icosa**
**83 avenue Denfert-Rochereau**
**75014 Paris (FR)**

(56) References cited:
**WO-A1-2016/075324     US-A1- 2012 071 780**

• **L. MAYAUD ET AL: "Robust Brain-computer Interface for virtual Keyboard (RoBIK): Project results", IRBM, vol. 34, no. 2, 1 April 2013 (2013-04-01), pages 131-138, XP055378375, AMSTERDAM, NL ISSN: 1959-0318, DOI: 10.1016/j.irbm.2013.01.013**
• **BARACHANT A ET AL: "Multiclass Brain Computer Interface Classification by Riemannian Geometry", IEEE TRANSACTIONS ON BIOMEDICAL ENGINEERING, IEEE SERVICE CENTER, PISCATAWAY, NJ, USA, vol. 59, no. 4, 1 April 2012 (2012-04-01), pages 920-928, XP011490017, ISSN: 0018-9294, DOI: 10.1109/TBME.2011.2172210**

**Description**

**FIELD OF INVENTION**

The present invention pertains to the field of signal processing. In particular, the invention relates to a system for computing a signal quality index from a multichannel bio-signal of a subject in order to detect artifacts.

**BACKGROUND OF INVENTION**

Bio-signals are a class of signals that are representations of underlying physiological processes. Bio-signals are particularly useful to monitor a patient's or subject's physiological state, diagnose illness or propose therapeutic or non-therapeutic methods.

A particularity of this class of signals is its high sensibility to environmental noise and other artifacts. Common artifacts are often of large amplitude compared to the bio-signal itself, which is typically a signal of rather small amplitude. Contamination by artifacts significantly reduces the utility of a bio-signal by giving a very low signal-to-noise ratio. During these artifacts, it is difficult to extract the information from noise. There is therefore a strong need in the medical field for signal processing techniques that remove artifacts from bio-signal prior to its interpretation.

One example of bio-signal is electroencephalogram, a measure of electrical brain activity. Electroencephalogram is very susceptible to various artifacts such as eye movement, eye blink, heart and muscle activities, electrode pop, swallowing, head and body movements...

A solution consists in detecting artifacts to suspend the processing: this approach avoids making bad signal processing. Said solution requires designing a quality index and a threshold for the detection of artifacts.

It is known to use covariance matrices which are processed with Riemannian geometry to detect artifacts from multichannel electroencephalogram signals (A. Barachant, A. Andreev, and M. Congedo, "The Riemannian potato: an automatic and adaptive artifact detection method for online experiments using Riemannian geometry", in Proceedings of TOBI Workshop IV, 2013, pp.19-20).

The method introduced by Barachant *et al.* computes the Riemannian distance between a current covariance matrix and an online covariance matrix mean. An arithmetic z-score of the distance is then computed. If the z-score is lower than a given threshold, the signal is considered as normal and covariance matrix mean is updated; if the z-score is higher than the threshold, the signal is considered as artifacted. The z-score may thus be considered as a signal quality index.

The method disclosed by Barachant *et al.* is applied on all the channels $C$ of the EEG signal, which give a feature space with $C \times \left( \frac{C+1}{2} \right)$. However, if the dimensions of the covariance matrix become too large, even a huge variation in one dimension (representative of an artifact) disappears in the noise of the other dimensions: the distance between the current covariance matrix and the reference covariance matrix does not increase significantly and does not exceed the threshold. Consequently, when the number of channels $C$ of the bio-signal is important (just as the number of dimensions of the covariance matrix), the detection of artifacts lacks efficiency. This phenomenon is accentuated with spatiofrequential covariance matrix: for $F$ frequency bands, it gives a feature space with $F \times C \times \left( \frac{C+1}{2} \right)$. Further relevant prior art is known from L. MAYAUD ET AL: "Robust Brain-computer Interface for virtual Keyboard (RoBIK): Project results",IRBM, vol. 34, no. 2, 1 April 2013 (2013-04-01), pages 131-138.

It is therefore an object of the present invention to provide an improved method for detecting all types of artifacts, even those occurring on a limited (e.g. one) number of channels among multichannel bio-signal.

**SUMMARY**

To that end, the present invention provides a system for computing a signal quality index from multichannel bio-signal of a subject wherein said multichannel bio-signal is divided into multiple selected bio-signals based on subsets of channels and/or on frequency bands dedicated to the detection of specific artifacts. Said method is more sensitive to the variation of each channel.

The present invention thus relates to a system for computing a signal quality index from a multichannel bio-signal of a subject, said system comprising:

- at least two sensors for acquiring one multichannel bio-signal of said subject on a current epoch $t$;
- a memory comprising at least one reference covariance matrix $\overline{\overline{\Sigma}}_t$, at least one first threshold and the mean and standard deviation of Riemannian distances from the previous epochs; and
- a computing unit for computing a signal quality index;

wherein the memory is connected to the computing unit and the at least two sensors are connected to the computing unit; and
wherein the computing unit:

- selects at least one subset of channels from said multichannel bio-signal or applies at least one frequency filtering to said multichannel bio-signal in order to get at least two selected bio-signals on the current epoch $t$;
- for each of said at least two selected bio-signals on the current epoch $t$:

  ▪ computes a current covariance matrix $\Sigma$;
  ▪ computes the current Riemannian distance d between said current covariance matrix $\Sigma$ and the at least one reference covariance matrix $\bar{\Sigma}_t$;
  ▪ computes an intermediary index based on a normalization of the current Riemannian distance $d$ by the mean and the standard deviation of the previous Riemannian distances obtained on the previous epochs for which the intermediary index was inferior to the first threshold; and

- computes a signal quality index based at least on the intermediary index for each of said at least two selected bio-signals.

According to one embodiment, the multichannel bio-signal is filtered in multiple frequency bands and concatenated by the computed unit and the computing unit computes a current spatiofrequential covariance matrix for each of said at least two selected bio-signals. According to one embodiment, the normalization for computing the intermediary index uses the geometric mean and the geometric standard deviation, more preferably the online geometric mean and the online geometric standard deviation. According to one embodiment, the normalization for computing the intermediary index is a z-score, preferably a geometric z-score.

According to one embodiment, for each of said at least two selected bio-signals on the current epoch t the computing unit updates the reference covariance matrix with the current covariance matrix if the intermediary index is inferior to the first threshold. According to one embodiment, the reference covariance matrix is updated as follows:

$$\bar{\Sigma}_{t+1} = \bar{\Sigma}_t^{\frac{1}{2}} \left( \bar{\Sigma}_t^{-\frac{1}{2}} \Sigma \, \bar{\Sigma}_t^{-\frac{1}{2}} \right)^{\alpha} \bar{\Sigma}_t^{\frac{1}{2}}$$

where $\alpha \in [0,1]$ is a coefficient defining the speed of adaptation; it can be a constant or chosen as $1/(t + 1)$. According to one embodiment, the multichannel bio-signal is an EEG signal. According to one embodiment, the multichannel bio-signal is an EEG signal and the computing unit selects a subset of channels representative of an EEG signal in a frontal area in order to get at least one selected bio-signal. According to one embodiment, the multichannel bio-signal is an EEG signal and the computing unit selects a subset of channels representative of two sensors in order to get at least one selected bio-signal. According to one embodiment, wherein the multichannel bio-signal is an EEG signal and the computing unit selects a subset of channels representative of an EEG signal in an occipital area in order to get at least one selected bio-signal. According to one embodiment, the multichannel bio-signal is an EEG signal and the computing unit selects a subset of channels representative of an EEG signal in a temporal area in order to get at least one selected bio-signal.

According to one embodiment, the at least one selected bio-signal is filtered by the computing unit in low frequencies, preferably in the frequency band ranging from 1 Hz to 20 Hz or in high frequencies, preferably in the frequency band ranging from 55 Hz to 95 Hz. According to one embodiment, at least one selected bio-signal is obtained by frequency filtering the multichannel bio-signal in at least one frequency band which is not of interest for the underlying physiological process, such as a frequency band ranging from 1 Hz to 3 Hz or from 32 Hz to 45 Hz.

According to one embodiment, the memory further comprises a second threshold and the computing unit identifies artifacts if the signal quality index is lower than the second threshold. According to one embodiment, the signal quality index is computed using Fisher's combined probability test, preferably a right-tailed Fisher's combined probability test.

The present invention also relates to a method for computing a signal quality index from a multichannel bio-signal of a subject comprising iteratively the following steps:

i. obtaining one multichannel bio-signal from the subject on a current epoch $t$;

ii. selecting a subset of channels from said multichannel bio-signal or applying a frequency filtering to said multichannel bio-signal in order to get at least two selected bio-signals on the current epoch $t$;

iii. for each of said at least two selected bio-signals on the current epoch $t$:

o computing a current covariance matrix $\Sigma$;

o computing the current Riemannian distance $d$ between said current covariance matrix $\Sigma$ and a reference covariance matrix $\bar{\bar{\Sigma}}_t$;

o computing an intermediary index based on a normalization of the current Riemannian distance d by the mean and standard deviation of the previous Riemannian distances obtained on the previous epoch for which the intermediary index was inferior to a first threshold;

o preferably updating the reference covariance matrix with the current covariance matrix if the intermediary index is inferior to a first threshold;

o preferably updating the mean and standard deviation of the previous Riemannian distances if the intermediary index is inferior to a first threshold;

iv. computing a signal quality index based at least on the intermediary index for each of said at least two selected bio-signals; and

v. repeating steps i) to iv).

**DEFINITIONS**

In the present invention, the following terms have the following meanings:

-  The term **"about"** is used herein to mean approximately, roughly, around, or in the region of. When the term "about" is used in conjunction with a numerical range, it modifies that range by extending the boundaries above and below the numerical values set forth. The term "about" is used herein to modify a numerical value above and below the stated value by a variance of 20 percent, preferably of 5 percent.

-  **"Bio-signal"** refers herein to any signal in subjects that can be continually measured and monitored. Bio-signal refers especially to any biological parameter which can be measured by an instrument which converts a physical measure (light, pressure, electricity, radio-signal...) into an analogous signal (in volts) and which is then digitalized.

-  **"Computing unit"** refers to a computer-based system or a processor-containing system or other system which can fetch and executes the instructions of a computer program.

-  **"Electrode"** or **"sensor"** refers to a conductor used to establish electrical contact with a nonmetallic part of a circuit. For instance, EEG electrodes are small metal discs usually made of stainless steel, tin, gold, silver covered with a silver chloride coating; they are placed on the scalp in specific positions.

-  **"Electroencephalogram"** or **"EEG"** refers to the record of the electrical activity of the brain of a subject, made by electrodes applied on the scalp.

-  **"Epoch"** or **"time-window"** refers to a determined period during which bio-signal is recorded. Epochs can be overlapped.

-  **"Fisher's combined probability test"** refers to a method used to combine p-value from several tests. See R. Fisher, Statistical methods for research workers, Genesis Publishing Pvt Ltd, 1925.

-  **"Index"** refers to any value obtained or computed according to the present invention. In the sense of the present invention, the index is a value understandable by a subject.

-  **"Neural signal"** refers herein to the bio-signal obtained by measuring neural activity. Said neural activity may be measured by: deep brain electrodes; electrocorticography (ECoG); electroencephalography (EEG); magnetoencephalography (MEG); magnetic resonance imaging (MRI): diffusion MRI, perfusion MRI, functional MRI (fMRI); near-infrared spectroscopy (NIRS); positron emission tomography (PET); or stereoelectroencephalography (SEEG).

- **"Normalization"** refers to a mathematical process to define values in an expected range: normalized values can be bounded, or normalized values can follow a normal/Gaussian distribution.

- **"Online"** refers to methods that work on a sample-by-sample basis. However, the EEG acquisition is generally carried out by buffered blocks. These methods are different from "block-online" in that the block size may be very small (typically, 10 to 50ms for online, instead of 1 to 4s for block-online).

- **"Riemannian manifold"** refers to a differentiable topological space that is locally homeomorphic to a Euclidean space, and over which a scalar product is defined that is sufficiently regular. The scalar product makes it possible to define a Riemannian geometry on the Riemannian manifold.

- **"Subject"** refers to a mammal, preferably a human. In the sense of the present invention, a subject may be a patient, i.e. a person receiving medical attention, undergoing or having underwent a medical treatment, or monitored for the development of a disease.

- **"Symmetric positive definite (SPD) matrix"** refers to a square matrix that is symmetrical about its diagonal, *i.e.* $\Sigma(i,j) = \Sigma(j,i)$, and that has eigenvalues that are strictly positive. An SPD matrix of dimensions $C \times C$ has $C \times \left( \frac{C+1}{2} \right)$ independent elements; it may therefore be locally approximated by a Euclidean space of $C \times \left( \frac{C+1}{2} \right)$ dimensions. It is possible to show that the SPD space has the structure of a Riemannian manifold. It is known that covariance matrices are symmetric positive definite matrices.

- **"Threshold"** refers to a reference value to which a current value is compared.

## DETAILED DESCRIPTION

This invention relates to a system for computing a signal quality index from a multichannel bio-signal of a subject.

The system comprises at least two sensors for acquiring one multichannel bio-signal of said subject.

According to one embodiment, the multichannel bio-signal is a multichannel neural signal.

According to one embodiment, the multichannel neural signal is a multichannel EEG signal. In said embodiment, various types of suitable headsets or electrodes systems or sensors systems are available for acquiring such a EEG signal. Examples includes, but are not limited to: Epoc headset commercially available from Emotiv, Mindset headset commercially available from Neurosky, Versus headset commercially available from SenseLabs, DSI 6 headset commercially available from Wearable sensing, Xpress system commercially available from BrainProducts, Mobita system commercially available from TMSi, Porti32 system commercially available from TMSi, ActiChamp system commercially available from BrainProducts and Geodesic system commercially available from EGI.

According to one embodiment, the multichannel EEG signal is acquired using a set of sensors (also referred to as electrodes). According to one embodiment, the multichannel EEG signal is acquired using 8 electrodes, 16 electrodes, 24 electrodes, 32 electrodes, 64 electrodes, 128 electrodes or 256 electrodes. Said electrodes are preferably positioned according to the international 10-20 system.

The overall acquisition time of the multichannel bio-signal is subdivided into periods, known in the art as epochs. Each epoch is associated with a matrix $X$ representative of the bio-signal acquired during said epoch.

Therefore, the system comprises at least two sensors for acquiring one multichannel bio-signal of said subject on successive epochs.

According to one embodiment, in order to ensure online (or real-time) processing, successive epochs are overlapped.

A multichannel bio-signal $X \in \mathbb{R}^{C \times N}$ is composed of $C$ channels, electrodes or sensors and $N$ time samples. For example, a subject is fitted with $C$ electrodes for neural signal acquisitions. Each electrode $c = 1 \dots C$ delivers a signal $X_c(n)$ as a function of time. The signal is sampled so as to operate in discrete time: $X(c,n) = X_c(n)$, and then digitized. This produced a matrix representation of the neural signal $X \in \mathbb{R}^{C \times N}$.

According to one embodiment, the multichannel bio-signal is pre-processed, such as for instance centered, filtered, cleaned and/or denoised.

The system further comprises a memory and a computing unit. The memory is connected to the computing unit. The at least two sensors are connected to the computing unit for delivering the multichannel bio-signal.

The computing unit is configured to obtain at least two selected bio-signals from the original multichannel bio-signal either by selecting a subset of channels from the multichannel bio-signal or by frequency filtering the multichannel bio-signal.

Consequently, the computing unit selects at least one subset of channels from said multichannel bio-signal or applies at least one frequency filtering to said multichannel bio-signal in order to get at least two selected bio-signals on the successive epochs.

According to one embodiment wherein the computing unit selects at least one subset of channels from said multichannel bio-signal, the selected bio-signal comprises one channel. According to one embodiment wherein the computing unit selects at least one subset of channels from said multichannel bio-signal, the selected bio-signal comprises a plurality of channels.

As explained hereabove, said selection and/or frequency filtering enables sensitive detection of specific artifacts.

According to an alternative embodiment, the computing unit is configured to obtain at least one selected bio-signal from the original multichannel bio-signal either by selecting a subset of channels from the multichannel bio-signal or by frequency filtering the multichannel bio-signal.

According to one embodiment wherein the multichannel bio-signal is an EEG signal, in order to detect electrode pops, the computing unit selects at least one subset of channel representative of two sensors. According to one embodiment wherein the multichannel bio-signal is an EEG signal, in order to detect electrode pop, the computing unit selects a plurality of selected bio-signals, each representative of two sensors. According to one embodiment wherein the multichannel bio-signal is an EEG signal, in order to detect electrode pop, the computing unit selects a selected bio-signal for each couple of two sensors.

According to one embodiment, said two sensors are:

- two adjacent sensors;
- two sensors symmetrical with respect to an axis, preferably with respect to an anteroposterior axis or a mediolateral axis, more preferably with respect to the anteroposterior axis or the mediolateral axis extending through the vertex; or
- two sensors symmetrical with respect to a third sensor acting as a center of symmetry, preferably two sensors symmetrical with respect to the vertex.

According to one embodiment, said selected bio-signals are further band-pass filtered, preferably in low frequencies. According to one embodiment, selected bio-signals are band-pass filtered from about 1 to about 20 Hz.

According to one embodiment wherein the multichannel bio-signal is an EEG signal, in order to detect electrooculogram activities such as for instance eye blinks, the computing unit selects a subset of channels representative of an EEG signal in a frontal area (i.e. at least one of the selected bio-signal comprises a subset of channels representative of frontal sensors). According to one embodiment, said selected bio-signal is further band-pass filtered, preferably in low frequencies. According to one embodiment, selected bio-signal is band-pass filtered from about 1 to about 20 Hz.

According to one embodiment wherein the multichannel bio-signal is an EEG signal, in order to detect muscular artifacts such as for instance neck tension, the computing unit selects a subset of channels representative of an EEG signal in occipital area (i.e. at least one of the selected bio-signal comprises a subset of channel representative of occipital sensors). According to one embodiment, said selected bio-signal is further band-pass filtered, preferably in high frequencies. According to one embodiment, selected bio-signal is band-pass filtered from about 55 to about 95 Hz or from about 65 to about 95 Hz or from about 65 to about 115 Hz.

According to one embodiment wherein the multichannel bio-signal is an EEG signal, in order to detect muscular artifacts such as for instance jaw clenching, the computing unit selects a subset of channels representative of a temporal EEG signal (i.e. at least one of the selected bio-signal comprises a subset of channels representative of temporal sensors). According to one embodiment, said selected bio-signal is further band-pass filtered, preferably in high frequencies. According to one embodiment, selected bio-signal is band-pass filtered from about 55 to about 95 Hz (if powerline is 50 Hz), or from about 65 to about 115 Hz (if powerline is 60 Hz).

According to one embodiment wherein the multichannel bio-signal is an EEG signal, in order to detect multiple artifacts such as for instance head motion, the computing unit frequency filters the multichannel bio-signal in at least one frequency band which is not of interest for the underlying process (for example, in at least one frequency band which is not the alpha band from 8 to 12 Hz or the beta band from 12.5 to 30 Hz). According to one embodiment, the computing unit frequency filters the multichannel bio-signal in a frequency band ranging from about 1 to about 3 Hz and/or in the frequency band ranging from about 32 to about 45 Hz.

According to one embodiment, the selected bio-signals comprise any combination of the above-described selected bio-signals.

According to one embodiment, the computing unit obtains 2, 3, 4, 5, 10, 15 or 20 selected bio-signals from any combination of the above-described embodiments.

According to one embodiment, for each of said at least two selected bio-signals on the current epoch *t* the computing unit:

- computes a current covariance matrix $\Sigma_t$, denoted $\Sigma$ in the following to lighten notations;
- computes the current Riemannian distance d between said current covariance matrix $\Sigma$ and a reference covariance matrix $\bar{\bar{\Sigma}}_t$; and
- computes an intermediary index based on a normalization of the current Riemannian distance $d$ by the mean and standard deviation of the previous Riemannian distances obtained on the previous epochs for which the intermediary index was inferior to a first threshold.

Said reference covariance matrix $\bar{\bar{\Sigma}}_t$, said first threshold and said mean and standard deviation of the Riemannian distances computed from previous epochs are stored in the memory.

According to one embodiment, the first threshold is ranging from 1.0 to 3.5, preferably 2.0. According to the Applicant, a low value provides a strict behavior with updates only on "very clean" epochs, a high value provides a more dynamic behavior, with more updates.

According to one embodiment, a first threshold is stored in the memory for each selected bio-signal.

According to one embodiment, a covariance matrix $\Sigma$ is computed from the bio-signal as follows:

$$\Sigma = \frac{1}{N-1} X^T X$$

, with $N \geq C$, where $N$ is the number of samples in the epoch for each electrode and $C$ the number of electrodes.

In another embodiment, the covariance matrix is computed using any method known by the skilled artisan, such as those disclosed in Barachant A. Commande robuste d'un effecteur par une interface cerveau-machine EEG asynchrone, PhD. Thesis, Université de Grenoble: FR, 2012.

According to one embodiment, the covariance matrix is a spatial covariance matrix. According to an alternative embodiment, the covariance matrix is a spatiofrequential covariance matrix. Said embodiment enables to be sensitive to specific frequency bands. Such sensitivity is of particular importance for neural signal, especially EEG signal.

According to one embodiment, the cut-off frequencies of the frequential filtering are chosen arbitrarily. According to another embodiment, the cut-off frequencies of the frequential filtering are optimized thanks to a preliminary frequential study.

According to one embodiment, in order to obtain a spatiofrequential covariance matrix, the bio-signal $X \in \mathbb{R}^{C \times N}$ is filtered in F frequency bands; thereby obtaining f = 1... F filtered signals $X_f \in \mathbb{R}^{C \times N}$. An extended signal $\widetilde{X} \in \mathbb{R}^{CF \times N}$ defined as the vertical concatenation of the filtered signals is computed as:

$$\widetilde{X} = \begin{bmatrix} X_1 \\ \vdots \\ X_f \\ \vdots \\ X_F \end{bmatrix}.$$

The spatiofrequential covariance matrix $\widetilde{\Sigma} \in \mathbb{R}^{CF \times CF}$ is then computed as follows: $\widetilde{\Sigma} = \frac{1}{N-1} \widetilde{X}^T \widetilde{X}$, with $N \geq CF$ where $N$ is the number of samples in the epoch for each electrode, and $C$ the number of electrodes.

According to one embodiment, the covariance matrix (either spatial or spatiofrequential) is normalized. According to one embodiment, the covariance matrix is trace-normalized, which makes its trace equal to 1:

$$\Sigma = \frac{\Sigma}{\text{trace}(\Sigma)}.$$

According to one embodiment, the covariance matrix is determinant-normalized, which makes its determinant equal to 1:

$$\Sigma = \frac{\Sigma}{\det(\Sigma)^{1/C}}.$$

The normalization step, especially the determinant normalization allows a session-to-session and/or a subject-to-subject transfer learning: it removes a part of variabilities from one recording to another.

Each covariance matrix associated with a given epoch is considered to be point of a Riemannian manifold. In order to position the neural activity of a subject relative to a reference, distance and means calculation are required. The metric used for covariance matrices has been detailed in Förstner W, Moonen B. A metric for covariance matrices. Quo vadis geodesia, pp. 113-128, 1999.

According to one embodiment, the Riemannian distance between two covariance matrices $\Sigma_1$ and $\Sigma_2$ may be defined as the affine-invariant distance:

$$d(\Sigma_1, \Sigma_2) = \left( \sum_{c=1}^{C} \log^2 \lambda_c(\Sigma_1, \Sigma_2) \right)^{\frac{1}{2}}$$

with $\lambda_c(\Sigma_1, \Sigma_2)$ the eigenvalues from $\det(\lambda\Sigma_1 - \Sigma_2) = 0$.

According to another embodiment, the Riemannian distance is computed using any other distances known by one skilled in the art, such as those described in Li Y, Wong KM. Riemannian Distances for Signal Classification by Power Spectral Density. IEEE Journal of selected topics in signal processing, vol.7, No.4, August 2013.

According to one embodiment, the Riemannian distances are estimated on the Riemannian manifold of symmetric positive definite matrices of dimensions equal to the dimensions of the covariance matrices.

According to one embodiment, the normalization for computing the intermediary index uses the mean $\mu_t$ and the standard deviation $\sigma_t$ of the previous Riemannian distances, preferably computed in their geometric form, for instance as follows:

$$\mu_t = \exp\left( \frac{1}{t} \sum_{\tau=1}^{t} \log(d_\tau) \right)$$

$$\sigma_t = \exp\left( \sqrt{\frac{1}{t} \sum_{\tau=1}^{t} \left( \log\left(\frac{d_\tau}{\mu_t}\right) \right)^2} \right)$$

According to one embodiment, mean $\mu_t$ and the standard deviation $\sigma_t$ can be online updated, for instance as follows:

$$\mu_{t+1} = \exp\left( \frac{t}{t+1} \log(\mu_t) + \frac{1}{t+1} \log(d_{t+1}) \right)$$

$$\sigma_{t+1} = \exp\left( \sqrt{\frac{t}{t+1} (\log(\sigma_t))^2 + \frac{1}{t+1} \left( \log\left(\frac{d_{t+1}}{\mu_{t+1}}\right) \right)^2} \right)$$

According to one embodiment, the intermediary index is defined as the normalization of the Riemannian distance providing a z-score, also called standard score.

According to one embodiment, the z-score is preferably computed in its geometric form, for instance as follows:

$$z_t = \frac{\log\left(\frac{d_t}{\mu_t}\right)}{\log(\sigma_t)}$$

According to one embodiment, for each of said at least two selected bio-signals on the current epoch $t$ the computing unit updates the reference covariance matrix with the current covariance matrix if the intermediary index is inferior to the first threshold.

According to one embodiment, the reference covariance matrix is updated as follows:

$$\bar{\Sigma}_{t+1} = \bar{\Sigma}_t^{\frac{1}{2}} \left( \bar{\Sigma}_t^{-\frac{1}{2}} \Sigma \bar{\Sigma}_t^{-\frac{1}{2}} \right)^{\frac{1}{t+1}} \bar{\Sigma}_t^{\frac{1}{2}}$$

According to one embodiment, update coefficient $\frac{1}{t+1}$ can be replaced by any other coefficient $\alpha \in [0,1]$ defining the speed of the adaptation.

As detailed hereabove, the reference covariance matrix is updated only if the intermediary index is lower than the first thresholds. Said embodiment prevents from updating the reference covariance matrix with a bio-signal comprising artifacts.

According to one embodiment, the reference covariance matrix is initialized online on the first epochs of the recording: $\bar{\Sigma}_{t=1} = \Sigma_1$ , $\mu_1 = 1$ , $\sigma_1 = e$. This type of online initialization provides an online dynamic process.

According to one embodiment, the reference covariance matrix is initialized offline during a calibration session, avoiding an online initialization on the first epochs, potentially containing artifacts due to the device installation and thus disturbing the efficiency of the detection. In this case, the initial value $t_0$ of $t$ defines the importance given to the calibration (high value for a confident initialization): $\bar{\bar{\Sigma}}_{t=t_0} = \bar{\Sigma}_{calibration}$ , $\mu_{t_0} = \mu_{calibration}$ , $\sigma_{t_0} = \sigma_{calibration}$. This type of offline initialization provides an online semi-static process, and is suitable for session-to-session or subject-to-subject transfer learning.

The computing unit further computes a signal quality index based at least on the intermediary index for each of said at least two selected bio-signals.

According to one embodiment, the signal quality index is based on the combination of intermediary indices computed for each of said at least two selected bio-signals.

According to one embodiment, the signal quality index is further based on a temporary index computed from the original multichannel bio-signal.

According to one embodiment, the signal quality index is computed using Fisher's combined probability test, preferably a right-tailed Fisher's combined probability test. According to one embodiment, the intermediary indices are combined in a single p-value with a right-tailed Fisher's combined probability test.

According to one embodiment, considering $i = 1 \ldots I$ intermediary indices, p-values $p_i$ associated to each z-score $z_i$ is defined as:

$$p_i = 1 - \mathrm{cdf}_{\mathcal{N}_{0,1}}(z_i)$$

wherein $\mathrm{cdf}_{\mathcal{N}_{0,1}}$ is the cumulative distribution function of the normal distribution $\mathcal{N}_{0,1}$ with mean equals to 0 and standard deviation equals to 1.

According to one embodiment, said p-values are then combined as follows:

$$s = -2 \sum_{i=1}^{I} \log(p_i) \sim \chi_{2I}^2$$

According to one embodiment, the signal quality index is finally computed as follows:

$$p = 1 - \mathrm{cdf}_{\chi_{2I}^2}(s)$$

wherein $\mathrm{cdf}_{\chi^2_{2I}}$ is the cumulative distribution function of the chi-square distribution $\chi^2_{2I}$ with 2*I* degrees of freedom.

According to another embodiment, intermediary indices can be combined in a signal quality index using the Stouffer's method; the Edgington method; or the squared z-scores method.

According to one embodiment, the memory further comprises a second threshold.

According to one embodiment, the computing unit identifies artifacts if the signal quality index is lower than the second threshold. According to one embodiment, the computing unit considers the signal as "clean" if the signal quality index is higher than the second threshold.

According to one embodiment, the second threshold is ranging from 0.001 and 0.05, preferably 0.01.

The present invention further relates to a method for computing a signal quality index from a multichannel bio-signal of a subject comprising iteratively the following steps:

  i. obtaining one multichannel bio-signal from the subject on a current epoch *t*;
  ii. selecting a subset of channels from said multichannel bio-signal or applying a frequency filtering to said multichannel bio-signal in order to get at least two selected bio-signals on the current epoch *t*;
  iii. for each of said at least two selected bio-signals on the current epoch *t*:

    ◦ computing a current covariance matrix $\Sigma_t$, denoted $\Sigma$ to lighten notations;
    ◦ computing the current Riemannian distance *d* between said current covariance matrix $\Sigma$ and a reference covariance matrix $\overline{\overline{\Sigma}}_t$;
    ◦ computing an intermediary index based on a normalization of the current Riemannian distance d by the mean and standard deviation of the previous Riemannian distances obtained on the previous epoch for which the intermediary index was inferior to a first threshold;
    ◦ preferably updating the reference covariance matrix $\overline{\overline{\Sigma}}_t$ with the current covariance matrix if the intermediary index is inferior to a first threshold;
    ◦ preferably updating the mean $\mu_t$ and standard deviation $\sigma_t$ of the previous Riemannian distances if intermediary index is inferior to a first channel; and

  iv. computing a signal quality index combining at least the intermediary index for each of said at least two selected bio-signals;
  v. repeating steps i) to iv).

According to one embodiment, the selection of a subset of channels from said multichannel bio-signal or the application of a frequency filtering to said multichannel bio-signal is implemented as detailed hereabove depending of the artifacts to be detected.

According to one embodiment, said method is a computer-implemented method.

According to one embodiment, the present invention also relates to computer program product comprising instructions which, when the program is executed by a computer, cause the computer to carry out the steps of the method according to the present invention. According to one embodiment, the present invention also relates to a computer-readable storage medium comprising instructions which, when executed by a computer, cause the computer to carry out the steps of the method of the present invention.

While various embodiments have been described, the detailed description is not to be construed as being limited hereto. Various modifications can be made to the embodiments by those skilled in the art without departing from the scope of the disclosure as defined by the claims.

**EXAMPLES**

The present invention is further illustrated by the following examples.

Example 1

In said example, multichannel EEG signal is recorded using 8 electrodes Fpz, F3, Fz, F4, C3, Cz, C4 and Pz positioned according to the 10-20 system.

In said example the following selected bio-signals are used:

In order to detect electrode pops, the computing unit obtains 4 selected bio-signals by selecting the following channels:

  - Fpz and Fz;

- F3 and F4;
- C3, C4; and
- Cz and Pz.

Said selected bio-signals comprises EEG signal recorded from adjacent electrodes. Said selected bio-signals are further filtered using a band-pass filter from 1 to 20 Hz.

In order to detect multiple artifacts such as for instance head motion, the computing unit obtains a selected bio-signal by frequency filtering all the channels in the frequency band ranging from 1 to 3 Hz and from 32 to 45 Hz.

Example 2

In said example, multichannel EEG signal is recorded using 8 electrodes Fpz, F3, Fz, F4, C3, Cz, C4 and Pz positioned according to the 10-20 system.

In said example the following selected bio-signals are used:
In order to detect eye blinks, and more generally all electro-oculogram activities the computing unit obtains a selected bio-signal by selecting the following channels: Fpz, F3, Fz and F4, representative of frontal electrodes. Said selected bio-signal is further filtered using a band-pass filter from 1 to 20 Hz.

In order to detect multiple artifacts such as for instance head motion, the computing unit obtains a selected bio-signal by frequency filtering all the channels in the frequency band ranging from 1 to 3 Hz and from 32 to 45 Hz.

Example 3

In said example, multichannel EEG signal is recorded using 8 electrodes Fpz, F3, Fz, F4, C3, Cz, C4 and Pz positioned according to the 10-20 system.

In said example the following selected bio-signals are used:
In order to detect electrode pops, the computing unit obtains 4 selected bio-signals by selecting the following channels:

- Fpz and Fz;
- F3 and F4;
- C3, C4; and
- Cz and Pz.

Said selected bio-signals comprises EEG signal recorded from adjacent electrodes. Said selected bio-signals are further filtered using a band-pass filter from 1 to 20 Hz.

In order to detect eye blinks, and more generally all electro-oculogram activities the computing unit obtains a selected bio-signal by selecting the following channels: Fpz, F3, Fz and F4, representative of frontal electrodes. Said selected bio-signal is further filtered using a band-pass filter from 1 to 20 Hz.

In order to detect muscular artifact, such as for instance jaw clenching the computing unit obtains a selected bio-signal by selecting the following channel: Fpz, F3, F4, C3 and C4 representative of electrodes closest to muscles. Said selected bio-signal is further filtered using a band-pass filter from 65 to 95 Hz.

In order to detect multiple artifacts such as for instance head motion, the computing unit obtains a selected bio-signal by frequency filtering all the channels in the frequency band ranging from 1 to 3 Hz and from 20 to 48 Hz.

**Claims**

1. A system for computing a signal quality index from a multichannel bio-signal of a subject, said system comprising:

    - at least two sensors for acquiring one multichannel bio-signal of said subject on a current epoch $t$;
    - a memory comprising at least one reference covariance matrix $\overline{\overline{\Sigma}}_t$, at least one first threshold and the mean and standard deviation of Riemannian distances from the previous epochs; and
    - a computing unit for computing a signal quality index;

    wherein the memory is connected to the computing unit and the at least two sensors are connected to the computing unit; and
    wherein the computing unit:

- selects at least a first subset of channels from said multichannel bio-signal and/or applies at least one frequency filtering to said multichannel bio-signal from said first subset in order to get at least a first selected bio-signal on the current epoch *t*;
- selects at least a second subset of channels from said multichannel bio-signal and/or applies at least one frequency filtering to said multichannel bio-signal from said second subset in order to get at least a second selected bio-signal on the current epoch *t*;
- for each of said at least the first and the second selected bio-signals on the current epoch *t*:

  - computes a current covariance matrix $\Sigma$;
  - computes the current Riemannian distance *d* between said current covariance matrix $\Sigma$ and the at least one reference covariance matrix $\overline{\Sigma}_t$;
  - computes an intermediary index based on a normalization of the current Riemannian distance *d* by the mean and the standard deviation of the previous Riemannian distances obtained on the previous epochs for which the intermediary index was inferior to the first threshold; and

- computes a signal quality index based at least on the intermediary index for each of said at least two selected bio-signals.

2. The system according to claim **1,** wherein the multichannel bio-signal is filtered in multiple frequency bands and concatenated by the computed unit and wherein the computing unit computes a current spatiofrequential covariance matrix for each of said at least two selected bio-signals.

3. The system according to claim **1** or claim **2,** wherein the normalization for computing the intermediary index uses the geometric mean and the geometric standard deviation, more preferably the online geometric mean and the online geometric standard deviation.

4. The system according to claim **3,** wherein the normalization for computing the intermediary index is a z-score, preferably a geometric z-score.

5. The system according to any one of claims **1** to **4,** wherein for each of said at least two selected bio-signals on the current epoch t the computing unit updates the reference covariance matrix with the current covariance matrix if the intermediary index is inferior to the first threshold.

6. The system according to claim **5,** wherein the reference covariance matrix is updated as follows:

$$\overline{\Sigma}_{t+1} = \overline{\Sigma}_t^{\frac{1}{2}} \left( \overline{\Sigma}_t^{-\frac{1}{2}} \Sigma \, \overline{\Sigma}_t^{-\frac{1}{2}} \right)^{\frac{1}{t+1}} \overline{\Sigma}_t^{\frac{1}{2}}$$

7. The system according to any one of claims **1** to **6,** wherein the multichannel bio-signal is an EEG signal and the computing unit selects a subset of channels representative of an EEG signal in a frontal area in order to get at least one selected bio-signal.

8. The system according to any one of claims **1** to **7,** wherein the multichannel bio-signal is an EEG signal and the computing unit selects a subset of channels representative of two sensors in order to get at least one selected bio-signal.

9. The system according to any one of claims **1** to **8,** wherein the multichannel bio-signal is an EEG signal and the computing unit selects a subset of channels representative of an EEG signal in an occipital area in order to get at least one selected bio-signal.

10. The system according to any one of claims **1** to **9,** wherein the multichannel bio-signal is an EEG signal and the computing unit selects a subset of channels representative of an EEG signal in a temporal area in order to get at least one selected bio-signal.

11. The system according to any one of claims **1** to **10,** wherein the at least one selected bio-signal is filtered by the computing unit in low frequencies, preferably in the frequency band ranging from 1 Hz to 20 Hz or in high frequencies, preferably in the frequency band ranging from 55 Hz to 95 Hz or from 65 to 115 Hz.

12. The system according to any one of claims **1** to **11**, wherein at least one selected bio-signal is obtained by frequency filtering the multichannel bio-signal in at least one frequency band which is not of interest for the underlying physiological process, such as a frequency band ranging from 1 Hz to 3 Hz or from 32 Hz to 45 Hz.

13. The system according to any one of claims **1** to **12**, wherein the memory further comprises a second threshold and the computing unit identifies artifacts if the signal quality index is lower than the second threshold.

14. The system according to any one of claims **1** to **13**, wherein the signal quality index is computed using Fisher's combined probability test, preferably a right-tailed Fisher's combined probability test.

15. A computer implemented method for computing a signal quality index from a multichannel bio-signal of a subject comprising iteratively the following steps:

   i. obtaining one multichannel bio-signal from the subject on a current epoch $t$;
   ii. selecting a subset of channels from said multichannel bio-signal or applying a frequency filtering to said multichannel bio-signal in order to get at least two selected bio-signals on the current epoch $t$;
   iii. for each of said at least two selected bio-signals on the current epoch $t$:

   ○ computing a current covariance matrix $\Sigma$;
   ○ computing the current Riemannian distance $d$ between said current covariance matrix $\Sigma$ and a reference covariance matrix $\bar{\bar{\Sigma}}_t$;
   ○ computing an intermediary index based on a normalization of the current Riemannian distance $d$ by the mean and standard deviation of the previous Riemannian distances obtained on the previous epoch for which the intermediary index was inferior to a first threshold; and

   iv. computing a signal quality index based at least on the intermediary index for each of said at least two selected bio-signals; and
   v. repeating steps i) to iv).

**Patentansprüche**

1. System zum Berechnen eines Signalqualitätsindex aus einem mehrkanaligen Bio-Signal einer Versuchsperson, wobei das System umfasst:

   - mindestens zwei Sensoren zum Erfassen eines mehrkanaligen Bio-Signals von der Versuchsperson zu einem aktuellen Zeitabschnitt $t$,
   - einen Speicher, umfassend mindestens eine Referenzkovarianzmatrix $\bar{\bar{\Sigma}}_t$, mindestens einen ersten Schwellenwert und den Durchschnitt und die Standardabweichung von Riemann'schen Abständen aus den vorherigen Zeitabschnitten; und
   - eine Berechnungseinheit zum Berechnen eines Signalqualitätsindex; wobei der Speicher mit der Berechnungseinheit verbunden ist und die mindestens zwei Sensoren mit der Berechnungseinheit verbunden sind; und

   wobei die Berechnungseinheit:

   - mindestens eine erste Untergruppe von Kanälen aus dem mehrkanaligen Bio-Signal auswählt und/oder mindestens eine Frequenzfilterung auf das mehrkanalige Bio-Signal von der ersten Untergruppe anwendet, um mindestens ein erstes ausgewähltes Bio-Signal zu dem aktuellen Zeitabschnitt $t$ zu bekommen;
   - mindestens eine zweite Untergruppe von Kanälen aus dem mehrkanaligen Bio-Signal auswählt und/oder mindestens eine Frequenzfilterung auf das mehrkanalige Bio-Signal von der zweiten Untergruppe anwendet, um mindestens ein zweites ausgewähltes Bio-Signal zu dem aktuellen Zeitabschnitt $t$ zu bekommen;
   - für jedes des mindestens einen und zweiten ausgewählten Bio-Signals zu dem aktuellen Zeitabschnitt $t$;

   ▪ eine aktuelle Kovarianzmatrix $\Sigma$ berechnet;
   ▪ den aktuellen Riemann'schen Abstand $d$ zwischen der aktuellen Kovarianzmatrix $\Sigma$ und der mindestens

einen Referenzkovarianzmatrix $\overline{\overline{\Sigma}}_t$ berechnet;

- einen zwischenliegenden Index auf Grundlage einer Normalisierung des aktuellen Riemann'schen Abstands $d$ durch den Durchschnitt und die Standardabweichung der vorherigen Riemann'schen Abstände, die zu den vorherigen Zeitabschnitten erhalten wurden, für die der zwischenliegende Index geringer war als der erste Schwellenwert, berechnet; und

- einen Signalqualitätsindex auf Grundlage des zwischenliegenden Index für jedes der mindestens zwei ausgewählten Bio-Signale berechnet.

2. System nach Anspruch **1**, wobei das mehrkanalige Bio-Signal in mehreren Frequenzbändern gefiltert und durch die Berechnungseinheit verkettet wird, und wobei die Berechnungseinheit eine aktuelle Raum-Frequenz-Kovarianzmatrix für jedes der mindestens zwei ausgewählten Bio-Signale berechnet.

3. System nach Anspruch **1** oder Anspruch **2**, wobei die Normalisierung zum Berechnen des zwischenliegenden Index den geometrischen Durchschnitt und die geometrische Standardabweichung verwendet, mehr bevorzugt den geometrischen Online-Durchschnitt und die geometrische Online-Standardabweichung.

4. System nach Anspruch **3**, wobei die Normalisierung zum Berechnen des zwischenliegenden Index ein z-Wert, vorzugsweise ein geometrischer z-Wert, ist.

5. System nach einem der Ansprüche **1** bis **4**, wobei für jedes der mindestens zwei ausgewählten Bio-Signale zu dem aktuellen Zeitabschnitt t die Berechnungseinheit die Referenzkovarianzmatrix mit der aktuellen Kovarianzmatrix aktualisiert, wenn der zwischenliegende Index geringer ist als der erste Schwellenwert.

6. System nach Anspruch **5**, wobei die Referenzkovarianzmatrix wie folgt aktualisiert wird:

$$\overline{\Sigma}_{t+1} = \overline{\Sigma}_t^{\frac{1}{2}} \left( \overline{\Sigma}_t^{-\frac{1}{2}} \Sigma \, \overline{\Sigma}_t^{-\frac{1}{2}} \right)^{\frac{1}{t+1}} \overline{\Sigma}_t^{\frac{1}{2}}$$

7. System nach einem der Ansprüche **1** bis **6**, wobei das mehrkanalige Bio-Signal ein EEG-Signal ist und die Berechnungseinheit eine Untergruppe von Kanälen auswählt, die für ein EEG-Signal in einem frontalen Bereich repräsentativ sind, um mindestens ein ausgewähltes Bio-Signal zu bekommen.

8. System nach einem der Ansprüche **1** bis **7**, wobei das mehrkanalige Bio-Signal ein EEG-Signal ist und die Berechnungseinheit eine Untergruppe von Kanälen auswählt, die für zwei Sensoren repräsentativ sind, um mindestens ein ausgewähltes Bio-Signal zu bekommen.

9. System nach einem der Ansprüche **1** bis **8**, wobei das mehrkanalige Bio-Signal ein EEG-Signal ist und die Berechnungseinheit eine Untergruppe von Kanälen auswählt, die für ein EEG-Signal in einem okzipitalen Bereich repräsentativ sind, um mindestens ein ausgewähltes Bio-Signal zu bekommen.

10. System nach einem der Ansprüche **1** bis **9**, wobei das mehrkanalige Bio-Signal ein EEG-Signal ist und die Berechnungseinheit eine Untergruppe von Kanälen auswählt, die für ein EEG-Signal in einem temporalen Bereich repräsentativ sind, um mindestens ein ausgewähltes Bio-Signal zu bekommen.

11. System nach einem der Ansprüche **1** bis **10**, wobei das mindestens eine ausgewählte Bio-Signal durch die Berechnungseinheit in niedrigen Frequenzen, vorzugsweise in dem Frequenzband, das von 1 Hz bis 20 Hz reicht, oder in hohen Frequenzen, vorzugsweise in dem Frequenzband, das von 55 Hz bis 95 Hz oder von 65 bis 115 Hz reicht, gefiltert wird.

12. System nach einem der Ansprüche **1** bis **11**, wobei mindestens ein ausgewähltes Bio-Signal durch Frequenzfilterung des mehrkanaligen Bio-Signals in mindestens einem Frequenzband erhalten wird, das für den zugrunde liegenden physiologischen Prozess nicht von Interesse ist, wie ein Frequenzband, das von 1 Hz bis 3 Hz oder von 32 Hz bis 45 Hz reicht.

**13.** System nach einem der Ansprüche **1** bis **12**, wobei der Speicher weiter einen zweiten Schwellenwert umfasst und die Berechnungseinheit Artefakte identifiziert, wenn der Signalqualitätsindex geringer ist als der zweite Schwellenwert.

**14.** System nach einem der Ansprüche **1** bis **13**, wobei der Signalqualitätsindex unter Verwendung von Fisher's kombiniertem Wahrscheinlichkeitstest berechnet wird, vorzugsweise einem rechtsseitigen Fisher's kombinierten Wahrscheinlichkeitstest.

**15.** Computerimplementiertes Verfahren zum Berechnen eines Signalqualitätsindex aus einem mehrkanaligen Bio-Signal einer Versuchsperson, die folgenden Schritte iterativ umfassend:

i. Erhalten eines mehrkanaligen Bio-Signals von der Versuchsperson zu einem aktuellen Zeitabschnitt $t$;
ii. Auswählen einer Untergruppe von Kanälen aus dem mehrkanaligen Bio-Signal oder Anwenden einer Frequenzfilterung auf das mehrkanalige Bio-Signal, um mindestens zwei ausgewählte Bio-Signale zu dem aktuellen Zeitabschnitt t zu bekommen;
iii. für jedes der mindestens zwei ausgewählten Bio-Signals zu dem aktuellen Zeitabschnitt $t$;

∘ Berechnen einer aktuellen Kovarianzmatrix $\Sigma$;
∘ Berechnen des aktuellen Riemann'schen Abstands $d$ zwischen der aktuellen Kovarianzmatrix $\Sigma$ und einer Referenzkovarianzmatrix $\overline{\overline{\Sigma}}_t$;
∘ Berechnen eines zwischenliegenden Index auf Grundlage einer Normalisierung des aktuellen Riemann'schen Abstands $d$ durch den Durchschnitt und die Standardabweichung der vorherigen Riemann'schen Abstände, die zu den vorherigen Zeitabschnitten erhalten wurden, für die der zwischenliegende Index geringer war als ein erster Schwellenwert; und

iv. Berechnen eines Signalqualitätsindex auf Grundlage von mindestens dem zwischenliegenden Index für jedes der mindestens zwei ausgewählten Bio-Signale; und
v. Wiederholen von Schritten i) bis iv).

**Revendications**

**1.** Système de calcul d'un indice de qualité de signal à partir d'un bio-signal multicanal d'un sujet, ledit système comprenant :

- au moins deux capteurs pour acquérir un bio-signal multicanal dudit sujet à une époque actuelle $t$ ;
- une mémoire comprenant au moins une matrice de covariance de référence $\overline{\overline{\Sigma}}_t$, au moins un premier seuil et la moyenne et l'écart type des distances riemanniennes des époques précédentes ; et
- une unité de calcul pour calculer un indice de qualité de signal ;

dans lequel la mémoire est connectée à l'unité de calcul et les au moins deux capteurs sont connectés à l'unité de calcul ; et
dans lequel l'unité de calcul :

- sélectionne au moins un premier sous-ensemble de canaux à partir dudit bio-signal multicanal et/ou applique au moins un filtrage fréquentiel audit bio-signal multicanal dudit premier sous-ensemble afin d'obtenir au moins un premier bio-signal sélectionné à l'époque actuelle $t$ ;
- sélectionne au moins un deuxième sous-ensemble de canaux à partir dudit bio-signal multicanal et/ou applique au moins un filtrage fréquentiel audit bio-signal multicanal dudit deuxième sous-ensemble afin d'obtenir au moins un deuxième bio-signal sélectionné à l'époque actuelle $t$ ;
- pour chacun desdits au moins un premier et un deuxième bio-signaux sélectionnés à l'époque actuelle $t$ :

∘ calculer une matrice de covariance actuelle $\Sigma$ ;
∘ calculer la distance riemannienne actuelle $d$ entre ladite matrice de covariance actuelle $\Sigma$ et la au moins une matrice de covariance de référence $\overline{\overline{\Sigma}}_t$ ;
∘ calculer un indice intermédiaire basé sur une normalisation de la distance riemannienne actuelle $d$ par la moyenne et l'écart-type des distances riemanniennes précédentes obtenues aux époques précédentes

**15**

pour lesquelles l'indice intermédiaire était inférieur au premier seuil ; et

◦ calculer un indice de qualité de signal basé au moins sur l'indice intermédiaire pour chacun desdits au moins deux bio-signaux sélectionnés.

2. Système selon la revendication **1**, dans lequel le bio-signal multicanal est filtré dans plusieurs bandes de fréquences et concaténé par l'unité de calcul et dans lequelle l'unité de calcul calcule une matrice de covariance spatio-fréquentielle actuelle pour chacun desdits au moins deux bio-signaux sélectionnés.

3. Système selon la revendication **1** ou la revendication **2**, dans lequel la normalisation pour le calcul de l'indice intermédiaire utilise la moyenne géométrique et l'écart type géométrique, plus préférablement la moyenne géométrique en ligne et l'écart type géométrique en ligne.

4. Système selon la revendication **3**, dans lequel la normalisation pour le calcul de l'indice intermédiaire est un z-score, de préférence un z-score géométrique.

5. Système selon l'une quelconque des revendications **1** à **4**, dans lequel pour chacun desdits au moins deux bio-signaux sélectionnés à l'époque actuelle t l'unité de calcul met à jour la matrice de covariance de référence avec la matrice de covariance actuelle si l'indice intermédiaire est inférieur au premier seuil.

6. Système selon la revendication **5**, dans laquelle la matrice de covariance de référence est mise à jour comme suit :

$$\overline{\Sigma}_{t+1} = \overline{\Sigma}_t^{\frac{1}{2}} \left( \overline{\Sigma}_t^{-\frac{1}{2}} \Sigma \, \overline{\Sigma}_t^{-\frac{1}{2}} \right)^{\frac{1}{t+1}} \overline{\Sigma}_t^{\frac{1}{2}}$$

7. Système selon l'une quelconque des revendications **1** à **6**, dans lequel le bio-signal multicanal est un signal EEG et l'unité de calcul sélectionne un sous-ensemble de canaux représentatif d'un signal EEG dans une zone frontale afin d'obtenir au moins un bio-signal sélectionné.

8. Système selon l'une quelconque des revendications **1** à **7**, dans lequel le bio-signal multicanal est un signal EEG et l'unité de calcul sélectionne un sous-ensemble de canaux représentatif de deux capteurs afin d'obtenir au moins un bio-signal sélectionné.

9. Système selon l'une quelconque des revendications **1** à **8**, dans lequel le bio-signal multicanal est un signal EEG et l'unité de calcul sélectionne un sous-ensemble de canaux représentatif d'un signal EEG dans une zone occipitale afin d'obtenir au moins un bio-signal sélectionné.

10. Système selon l'une quelconque des revendications **1** à **9**, dans lequel le bio-signal multicanal est un signal EEG et l'unité de calcul sélectionne un sous-ensemble de canaux représentatif d'un signal EEG dans une zone temporale afin d'obtenir au moins un bio-signal sélectionné.

11. Système selon l'une quelconque des revendications **1** à **10**, dans lequel le au moins un bio-signal sélectionné est filtré par l'unité de calcul dans les basses fréquences, de préférence dans la bande de fréquence allant de 1 Hz à 20 Hz ou dans les hautes fréquences, de préférence dans la bande de fréquence allant de 55 Hz à 95 Hz ou de 65 à 115 Hz.

12. Système selon l'une quelconque des revendications **1** à **11**, dans lequel au moins un bio-signal sélectionné est obtenu par filtrage fréquentiel du bio-signal multicanal dans au moins une bande de fréquence sans intérêt pour le processus physiologique sous-jacent, comme une bande de fréquence allant de 1 Hz à 3 Hz ou de 32 Hz à 45 Hz.

13. Système selon l'une quelconque des revendications **1** à **12**, dans lequel la mémoire comprend en outre un deuxième seuil et l'unité de calcul identifie des artefacts si l'indice de qualité du signal est inférieur au deuxième seuil.

14. Système selon l'une quelconque des revendications **1** à **13**, dans lequel l'indice de qualité du signal est calculé à l'aide du test de probabilité combinée de Fisher, de préférence un test de probabilité combinée de Fisher unilatéral à droite.

**15.** Procédé mise en œuvre par ordinateur pour le calcul d'un indice de qualité du signal à partir d'un bio-signal multicanal d'un sujet, comprenant les étapes itératives suivantes :

i. obtention d'un bio-signal multicanal du sujet à l'époque actuelle $t$ ;
ii. sélection d'un sous-ensemble de canaux à partir dudit bio-signal multicanal ou application d'un filtrage fréquentiel audit bio-signal multicanal afin d'obtenir au moins deux bio-signaux sélectionnés à l'époque actuelle ;
iii. pour chacun desdits au moins deux bio-signaux sélectionnés à l'époque actuelle $t$ :

○ calculer une matrice de covariance actuelle $\Sigma$ ;
○ calculer la distance riemannienne actuelle $d$ entre ladite matrice de covariance actuelle $\Sigma$ et une matrice de covariance de référence $\overline{\overline{\Sigma}}_t$ ;
○ calculer un indice intermédiaire basé sur une normalisation de la distance riemannienne actuelle $d$ par la moyenne et l'écart type des distances riemanniennes précédentes obtenues à l'époque précédente pour laquelle l'indice intermédiaire était inférieur à un premier seuil ; et

iv. calcul d'un indice de qualité du signal basé au moins sur l'indice intermédiaire pour chacun desdits au moins deux bio-signaux sélectionnés ;
v. répétition des étapes i) à iv).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. BARACHANT ; A. ANDREEV ; M. CONGEDO.** The Riemannian potato: an automatic and adaptive artifact detection method for online experiments using Riemannian geometry. *Proceedings of TOBI Workshop IV,* 2013, 19-20
- **L. MAYAUD et al.** Robust Brain-computer Interface for virtual Keyboard (RoBIK): Project results. *IRBM,* 01 April 2013, vol. 34 (2), 131-138
- **R. FISHER.** Statistical methods for research workers. Genesis Publishing Pvt Ltd, 1925

- Commande robuste d'un effecteur par une interface cerveau-machine EEG asynchrone. **BARACHANT A.** PhD. Thesis. Université de Grenoble, 2012
- **FÖRSTNER W ; MOONEN B.** A metric for covariance matrices. *Quo vadis geodesia,* 1999, 113-128
- **LI Y ; WONG KM.** Riemannian Distances for Signal Classification by Power Spectral Density. *IEEE Journal of selected topics in signal processing,* August 2013, vol. 7 (4